Europäisches Patentamt

European Patent Office

Office européen des brevets

(11) Veröffentlichungsnummer: **0 438 641 A2**

# EUROPÄISCHE PATENTANMELDUNG

(21) Anmeldenummer: 90119868.9

(22) Anmeldetag: 17.10.90

(51) Int. Cl.5: **C07C 37/14, C07C 39/06**

(30) Priorität: 15.12.89 DE 3941472

(43) Veröffentlichungstag der Anmeldung:
31.07.91 Patentblatt 91/31

(84) Benannte Vertragsstaaten:
BE CH DE FR GB IT LI NL

(71) Anmelder: **HÜLS AKTIENGESELLSCHAFT**
**Patentabteilung / PB 15 - Postfach 13 20**
**W-4370 Marl 1(DE)**

(72) Erfinder: **Küpper, Friedrich-Wilhelm, Dr.**
**Oderbruchstrasse 27**
**W-4370 Marl(DE)**
Erfinder: **Müller, Wolfgang H.E., Dr.**
**Kaspar-Grove-Strasse 32**
**W-4370 Marl(DE)**
Erfinder: **Oberholz, Alfred, Dr.**
**Riegestrasse 64**
**W-4370 Marl(DE)**

(54) **Verfahren zur Herstellung von 2,6-Di-tert.butylphenol.**

(57)
2.1 Nach den Verfahren gemäß dem Stand der Technik wird DTBP durch Anlagerung von Isobuten an Phenol in Gegenwart von Aluminiumphenolaten oder durch Isobuten-Anlagerung an 2-Tert.butylphenol (2-TBP) mit Aluminium-tris-2-tert.butylphenolat als Katalysator hergestellt. Auch kombinierte Verfahren sind bekannt. Nachteilig sind die erforderlichen, relativ großen Katalysatormengen, die vor der destillativen Trennung der Reaktionsprodukte desaktiviert werden müssen und durch die Abwasserprobleme entstehen. Weiterhin nachteilig ist das Entstehen unerwünschter di- und trialkylierter Produkte.

Aufgabe der Erfindung war es, ein verbessertes, umweltfreundliches Verfahren zu entwickeln, das die genannten Nachteile nicht aufweist und zudem gute Ausbeuten bringt.

2.2 Die Lösung besteht darin, daß, ausgehend von 2-TBP die Umsetzung mit Isobuten in der Flüssigphase, katalysiert durch Aluminium-tris-(2-tert.butylphenolat), in Gegenwart von größeren Mengen unter Reaktionsbedingungen flüssigen gesättigten (cyclo)aliphatischen Kohlenwasserstoffen und/oder $C_5$-$C_{16}$-Alkenen der Formeln $R_1$-CH=$CH_2$ oder $R_2$-CH=CH-$R_3$ und/oder $C_5$- bis $C_{12}$-Cycloalkenen, die keine Verzweigungen an der C=C-Bindung enthalten, und/oder von überschüssigem Isobuten als Verdünnungsmittel bei Temperaturen von 0 °C bis 80 °C, Drücken von 0,1 bis 11 bar und Katalysatormengen von 0,005 bis 5 mol-%, bezogen auf eingesetztes 2-TBP, durchgeführt wird.

2.3 Aus DTBP können Antioxidantien und andere Verbindungen hergestellt werden.

EP 0 438 641 A2

# VERFAHREN ZUR HERSTELLUNG VON 2,6-DI-TERT.BUTYLPHENOL

Die Erfindung betrifft ein verbessertes Verfahren zur Herstellung von 2,6-Di-tert.butylphenol mit erhöhter Selektivität bei möglichst geringem Druck und möglichst niedrigen Temperaturen durch Umsetzung von 2-Tert.butylphenol mit Isobuten unter Katalyse mit Aluminiumtris-(2-tert.butylphenolat) in Gegenwart von gesättigten aliphatischen und/oder cycloaliphatischen Kohlenwasserstoffen und/oder überschüssigem flüssigem bzw. gelöstem Isobuten und/oder $C_5$- bis $C_{12}$-Cycloalkenen, die keine Verzweigungen an der C = C-Bindung enthalten, und/oder Alkenen der Formeln $R_1$-CH = $CH_2$ (1-Alkenen) bzw. $R_2$-CH = CH-$R_3$ mit 5 bis 16 C-Atomen.

Es ist bekannt, daß 2,6-Di-tert.butylphenol durch Anlagerung von Isobuten an Phenol oder an 2-Tert.butylphenol in Gegenwart von Aluminiumphenolaten gewonnen werden kann (vgl. Ullmann, "Enzyklopädie der Technischen Chemie", Band 18 (1979) Seite 205 ff.). Üblicherweise wird die Alkylierung von Phenol bzw. 2-Tert.butylphenol in Gegenwart von Aluminiumphenolat bei mindestens 100 °C, meist bei 110 °C bis 120 °C, unter erhöhtem Druck durchgeführt, der bis zu 25 bar betragen soll. Der Katalysator wird durch Lösen von 1 bis 3 Gew.-% Aluminium im zu alkylierenden Phenol gewonnen [vgl. US-PS 2 831 898, DE-PSS 944 014, 1 044 825; J. Org. Chem. 22 (1957) 642; Ang. Chem. 69 (1957) 699]. Er kann aber auch durch Umsetzung von Aluminiumalkoholaten oder von Aluminiumtriethyl mit Phenol sowie auf weiteren Wegen hergestellt werden. - In Abhängigkeit vom jeweils angewandten Phenol zu Isobuten-Verhältnis werden vorzugsweise 2-Tert.butylphenol oder 2,6-Di-tert.butylphenol erhalten. 2,4-Di-tert.butylphenol und 2,4,6-Tri-tert.butylphenol treten als Nebenprodukte auf. Die Verwendung eines Überschusses an Isobuten soll mit hohen Ausbeuten zur Bildung des trisubstituierten Phenols führen [vgl. Ullmann, "Enzyklopädie der Technischen Chemie", Band 18 (1979), S. 200; Kirk-Othmer, "Encyclopedia of Chemical Technology", Vol. 2 (1978), S. 85)]. Auch mit zunehmender Reaktionszeit soll der Anteil der einen para-ständigen Tert.butylsubstituenten enthaltenden Verbindungen (2,4-Di-tert.butylphenol, 2,4,6-Tri-tert.butylphenol) ansteigen. Daher ist die Reaktion in einem hinsichtlich der 2,6-Di-tert.butylphenolherstellung optimalen Zeitpunkt zu unterbrechen. Nach Abschluß der Alkylierung ist der Aluminiumphenolatkatalysator spätestens vor Beginn der destillativen Trennung der Reaktionsprodukte zu desaktivieren, um sonst zu beobachtende De- und Umalkylierungen zu vermeiden. Im allgemeinen erfolgt diese Desaktivierung durch Hydrolyse mit Wasser, Säure oder verdünnter Lauge, doch müssen selbst Spuren von Säuren vor der Destillation wieder entfernt oder neutralisiert werden. Dies "erfordert (vgl. Ullmann, S. 202) ein sorgfältiges Auswaschen des Alkylats mit Alkalilaugen und Wasser, wobei Abwässer anfallen, die (nach Abtrennen der wäßrigen Phase) nachbehandelt werden müssen", um Belastungen der Umwelt zu vermeiden. Die Rektifikation der nach diesem Herstellungsverfahren erhaltenen organischen Phase soll beispielsweise bei Einsatz von 2 Mol Isobuten pro Mol Phenol ca. 75 % d. Th. 2,6-Di-tert.butylphenol neben ca. 10 % d. Th. 2-Tert.butylphenol liefern. Der Destillationsrückstand soll im wesentlichen aus 2,4,6-Tri-tert.butylphenol bestehen. Setzt man andererseits nur 1 Mol Isobuten pro Mol Phenol ein, so isoliert man neben nicht umgesetztem Phenol und etwas 2,6-Di-tert.butylphenol als Hauptprodukt 2-Tert.butylphenol, das in etwa 70%iger Ausbeute, bezogen auf umgesetztes Phenol, anfallen soll.

Stellt man den Katalysator aus aluminiumorganischen Verbindungen, vorzugsweise Aluminiumtrialkylen, und 2-Tert.butylphenol an Stelle von Phenol her, so gelingt die Isobutenanlagerung an 2-Tert.butylphenol auch bei Temperaturen unterhalb von 100 °C (vgl. US-PS 3 355 504). Die Alkylierung soll trotz der niedrigeren Reaktionstemperaturen rasch zum gewünschten 2,6-Di-tert.butylphenol führen und diese Verbindung bei Verwendung vergleichbarer Katalysatormengen (ca. 1 - 3 mol-% bezogen auf eingesetztes 2-Tert.butylphenol) mit geringeren Anteilen unerwünschter Beimengungen (wie 2,4-Di-tert.butylphenol oder vor allem 2,4,6-Tri-tert.butylphenol) liefern. Auch dieses spezielle Katalysatorsystem ist vor Beginn der Aufarbeitung zu desaktivieren, um De- und Umalkylierungen während der destillativen Trennung der Reaktionsprodukte zu vermeiden.

Unabhängig von der Art des benutzten aluminiumhaltigen Katalysators wird die Gewinnung von 2,6-Di-tert.butylphenol durch Alkylierung von Phenol oder 2-Tert.butylphenol nach den Verfahren des Standes der Technik stets ohne Verwendung von Lösungsmitteln durchgeführt [vgl. Ullmann, "Enzyklopädie der Technischen Chemie", Band 18 (1979), S. 202]. Das Isobuten wird dabei so schnell zudosiert, wie es die Wärmeabfuhr aus dem Reaktor erlaubt. Dies ist notwendig, da die genaue Einhaltung eines engen Temperaturbereiches Voraussetzung für die Herstellung von 2,6-Di-tert.butylphenol mit den angegebenen Ausbeuten (von ca. 75 % d. Th.) bei Verwendung des Aluminium-tris-(phenolat)-katalysators ist (vgl. Ullmann, S. 205/206). Dieser wird offensichtlich bisher überwiegend verwandt. Andererseits ist auch bei der Alkylierung von 2-Tert.butylphenol in Gegenwart des Aluminium-tris-(2-tert.butylphenolat)-katalysators binnen weniger Minuten nach Zusatz des Isobutens ein erheblicher Temperaturanstieg zu beobachten [vgl. US-PS 3 355 504, Beispiele 2 und 3], der die Herstellung von 2,6-Di-tert.butylphenol bei konstanter niedriger Reaktionstemperatur erschwert und im größeren Maßstab evtl. unmöglich macht.

Nachteilig bei den älteren Verfahren des Standes der Technik sind auch die erforderlichen relativ großen Mengen des nur mäßig aktiven Aluminium-tris-(phenolat)-katalysators, die ebenso wie der Aluminium-tris-(2-tert.butylphenolat)-katalysator vor der destillativen Trennung der Reaktionsprodukte desaktiviert werden müssen. Die Beseitigung des dabei anfallenden, Aluminiumverbindungen und (Alkyl-)-Phenole enthaltenden Abwassers ist aus Gründen des Umweltschutzes unbedingt erforderlich, aber nicht unproblematisch. Dies deutet schon die Zahl der Patentanmeldungen an, die sich bis in die jüngste Zeit mit der Desaktivierung der Katalysatoren sowie mit teilweise recht aufwendigen Lösungen bei der Entsorgung des Abwassers bzw. mit der Verringerung der anfallenden Menge befassen (vgl. z. B. US-PS 3 200 157, DE-PS 1 809 555, DE-OS 2 039 062, US-PS 3 939 215, DE-PS 2 602 149, BE-PS 842 691, US-PS 3 652 685, US-PS 3 970 708). Die mit der Abwasserbeseitigung verbundenen Probleme waren bisher auch durch die Heterogenisierung bekannter Katalysatoren nicht zu lösen (vgl. EP-PS 0 206 085), da die katalytisch wirksamen Aluminiumphenolatkatalysatoren mit den Reaktionsprodukten in nicht zu vernachlässigendem Umfang ausgetragen werden.

Ein weiterer Nachteil der Verfahren des Standes der Technik besteht in der Bildung eines hohen Anteils an unerwünschten di- und vor allem trialkylierten Produkten bei Verwendung des Aluminium-tris-(phenolat)-katalysators zur Isobutenanlagerung an Phenol. Abtrennung und destillative Reinigung des gewünschten 2,6-Di-tert.butylphenols werden dadurch erheblich erschwert, andererseits wird ein nicht zu vernachlässigender Anteil an Einsatzmaterial in nicht ohne weiteres verwertbare Produkte überführt. Das Verfahren der US-PS 3 355 504 erlaubt es, den Anteil an 2,4,6-Tri-tert.butylphenol erheblich zu senken und 2,6-Di-tert.butylphenol mit Ausbeuten von ca. 93 % d. Th. herzustellen. Auch bei diesem Verfahren ist jedoch die Abführung der Reaktionswärme und damit die Einhaltung einer vorgegebenen Temperatur bei Verwendung der in den Beispielen 2 und 3 angegebenen Katalysatormengen offensichtlich ähnlich schwierig wie beim konventionellen Verfahren. Bei diesem muß - wie bereits erwähnt - die Isobutenzufuhr ggf. der Wärmeabführung angepaßt werden, um die Reaktionstemperatur im erforderlichen Bereich zu halten, und schließlich nach Erreichen eines optimalen 2,6-Di-tert.butylphenolanteils rasch ganz unterbrochen werden, um die Bildung größerer Anteile des unerwünschten 2,4,6-Tri-tert.butylphenols zu verhindern. Dieses beeinflußt die Wirtschaftlichkeit der herkömmlichen Verfahren so stark, daß auch seine Rückführung in den Alkylierungsprozeß unter erheblichem technischen Aufwand und trotz eines nur bescheidenen Anstiegs der 2,6-Di-tert.butylphenol-ausbeuten empfohlen wurde (vgl. US-PS 4 560 809). Das bei ca. 100 °C durchgeführte Verfahren erfordert im übrigen sowohl technische Einrichtungen zum Aufheizen des Reaktors bei der Katalysatorformierung (aus Aluminium) und beim Beginn der Alkylierung als auch leistungsfähige Kühleinrichtungen zur Temperaturkontrolle während der Reaktion.

Da die bekannten Verfahren des Standes der Technik die geschilderten Nachteile aufweisen, bestand die Aufgabe der Erfindung darin, ein verbessertes umweltfreundliches Verfahren zur Herstellung von 2,6-Di-tert.butylphenol mit erhöhten Ausbeuten bei geringem Katalysatorbedarf sowie verringertem Anteil an 2,4,6-Tri-tert.butylphenol und 2,4-Di-tert.butylphenol zu entwickeln.

Die Lösung dieser Aufgabe gelang in einer geringen technischen Aufwand erfordernden Weise, in dem 2,6-Di-tert.butylphenol durch Anlagerung von Isobuten an 2-Tert.butylphenol unter Katalyse mit Aluminium-tris-(2-tert.butylphenolat) in Gegenwart ausgewählter aliphatischer und/oder cycloaliphatischer Kohlenwasserstoffe und/oder von $C_5$- bis $C_{12}$-Cycloalkenen, die keine Verzweigungen an der $C=C$-Bindung enthalten, und/oder von Alkenen der Formeln $R_1-CH=CH_2$ (1-Alkenen) bzw. $R_2-CH=CH-R_3$ mit 5 bis 16 C-Atomen und/oder von überschüssigem flüssigem bzw. gelöstem Isobuten bei möglichst niedrigen Temperaturen und unter Verwendung möglichst geringer Katalysatormengen hergestellt wird.

Gegenstand der Erfindung ist daher ein Verfahren zur Herstellung von 2,6-Di-tert.butylphenol durch Umsetzung von 2-Tert.butylphenol mit Isobuten in der Flüssigphase in Gegenwart von Aluminium-tris-(2-tert.butylphenolat) als Katalysator, das dadurch gekennzeichnet ist, daß man die Umsetzung in Gegenwart von gesättigten aliphatischen und/oder cycloaliphatischen Kohlenwasserstoffen und/oder von überschüssigem flüssigem bzw. gelöstem Isobuten und/oder $C_5$- bis $C_{16}$-Alkenen der Formeln $R_1-CH=CH_2$ oder $R_2-CH=CH-R_3$, in denen $R_2$ und $R_3$ auch unter Bildung eines $C_5$- bis $C_{12}$-Cycloalkens verknüpft sein können, als Verdünnungsmittel bei Temperaturen von 0 °C bis 80 °C, bei Drucken von 0,1 bis 11 bar und mit Katalysatormengen von 0,005 bis 5 mol-%, bezogen auf eingesetztes 2-Tert.butylphenol, durchführt.

Diese Lösung ist außerordentlich überraschend, da nur der Zusatz der genannten Lösemittel in beachtlicher Menge oder noch überraschender eines Überschusses an flüssigem bzw. gelöstem Isobuten unter den genannten erfindungsgemäßen Bedingungen zu den gewünschten Verbesserungen hinsichtlich des Anteils an 2,4-Di-tert.butylphenol und vor allem an 2,4,6-Tri-tert.butylphenol führt.

Auch in der US-PS 3 355 504 wird auf die Nachteile eines hohen Gehalts an 2,4,6-Tri-tert.butylphenol ausdrücklich hingewiesen, und die gegenüber dem Stand der Technik (z. B. dem Verfahren der US-PS 2 831 898) erreichten Vorteile werden hervorgehoben. So wird in der US-PS 3 355 504 beispielsweise empfohlen, die Alkylierung von Phenol mit Isobuten in Gegenwart von Aluminium-tris(phenolat) oberhalb von 90 °C bei ca. 7 bar durchzuführen, bis der Gehalt an 2-Tert.butylphenol und 2,6-Di-tert.butylphenol einem Maximum zustrebt, während sich andererseits erst ein kleiner Anteil von maximal 5 bis 6 Prozent des unerwünschten 2,4,6-Tri-tert.butylphenols gebildet hat. Dann soll nach US-PS 3 355 504 (vgl. Sp. 3, Z. 10 ff.) die Reaktionstemperatur auf unter 90 °C abgesenkt werden, ehe die Reaktion nach Zusatz bzw. Formierung eines Aluminium-(2-alkylphenolat)-katalysators bei tieferer Temperatur fortzuführen ist. Dabei ist zunächst eine zum unumgesetzten Phenol und evtl. anwesenden (Phenol-)Ethern äquivalente stöchiometrische Menge an reaktivem "Aluminiumsalz" zuzusetzen, die mit dem Phenol unter Bildung von Aluminiumphenolat und mit den (Phenol-)Ethern unter Komplexbildung reagiert (vgl. Sp. 3, Z. 70 ff. und Sp. 4, Z. 1 ff.), ehe ein Überschuß von 0,001 bis 5,0 mol-%, vorzugsweise von 0,001 bis 1 mol-%, bezogen auf vorhandenes 2-Tert.butylphenol, an reaktivem "Aluminiumsalz" die weitere katalytische Umsetzung bewirkt. Ein größerer Überschuß an reaktiver Aluminiumverbindung, d. h. Anteile von bis zu 50 mol-% und mehr, ist zwar an sich überflüssig, kann aber nach Angaben der US-PS 3 355 504 ebenfalls ohne Nachteil zugesetzt werden. Die Aluminiumverbindungen können als solche, aber auch in Form von Lösungen in inerten Lösemitteln wie Hexan, Benzol oder Toluol zum Reaktionsgemisch zugesetzt werden. Die Lösemittel werden dabei nur in kleinen Mengen verwendet (vgl. Sp. 6, Z. 8 ff.). Die Isolierung von Aluminium-tris-(2-tert.butylphenolat) aus hexanhaltigem 2-Tert.butylphenol und das Waschen des abfiltrierten Niederschlags mit n-Hexan werden beschrieben.

Die Erfassung des Zeitpunktes, zu dem nach der bevorzugten zweistufigen Ausführungsform der US-PS 3 355 504 der anzustrebende optimale Gehalt von 75 - 100 mol-% bzw. 85 - 100 mol-% 2-Tert.butylphenol und 2,6-Di-tert.butylphenol erreicht ist, ehe nach Absenkung der Temperatur auf weniger als 90 °C und vorzugsweise auf weniger als 60 °C die Reaktion nach Formierung des 2-Tert.butylphenolatreste enthaltenden Katalysators fortgesetzt wird, ist schwierig und ebenso wie das schnelle Abkühlen des Reaktionsgemisches auf die erforderliche niedrigere Temperatur und die Festlegung der zuzusetzenden notwendigen minimalen Menge an reaktivem "Aluminiumsalz" (Aluminiumalkylen) sicher mit einem erheblichen technischen und analytischen Aufwand verbunden. Um den Fortgang der Alkylierung unter allen Umständen zu gewährleisten, muß daher ein Zusatz eines größeren Überschusses an Aluminiumalkylen, mindestens aber von Mengen in der Nähe der oberen Grenze des erwähnten optimalen Bereichs (von 0,001 bis 1 mol-%) erfolgen.

Dies hat zur Folge, daß bei der Aufarbeitung derartiger Reaktionsgemische erhebliche Mengen aluminiumhaltiger Katalysatoren bzw. Komplexe ohne Belastung der Umwelt entsorgt werden müssen, da zusätzlich zum zunächst bei > 90 °C benutzten Aluminium-tris-phenolat auch der bei niedrigerer Temperatur wirksame Katalysator und zusätzlich die zuvor gebildeten Reaktionsprodukte aus Phenol, (Phenol-)Ethern und Aluminiumalkylen anfallen. Angesichts der im Stand der Technik geschilderten Bemühungen um Verbesserungen bei der notwendigen Katalysatordesaktivierung und -beseitigung dürften diese Schritte beim in der US-PS 3 355 504 bevorzugten zweistufigen Verfahren heute hohe Kosten verursachen, die den Vorteil der verringerten 2,4,6-Tri-tert.butylphenolanteile zumindestens teilweise wieder kompensieren würden.

Bei dem einstufigen Verfahren nach US-PS 3 355 504 verläuft die Isobutenanlagerung an 2-Tert.butylphenol in Gegenwart der in Beispiel 2 und 3 verwandten Katalysatormengen rasch und - trotz der eingesetzten nur kleinen Menge an 2-Tert.butylphenol - unter starker Wärmeentwicklung. Katalysatorformierung und Alkylierung erfolgen in Anwesenheit von etwas Toluol. Die beobachteten Anteile an Nebenprodukten der 2,6-Di-tert.butylphenolsynthese, wie z. B. 2,4,6-Tri-tert.butylphenol, wurden nicht angegeben. Ebenso wie der Beschreibung ist auch den Beispielen der Patentschrift keine Lehre zu entnehmen, ob ein derartiges Verfahren problemlos in einen größeren Maßstab übertragen werden kann und ob dabei 2,4,6-Tri-tert.butylphenol nur in geringer Menge gebildet wird.

Angesichts der geschilderten Nachteile und der in Teilbereichen unklaren Lehren des dem erfindungsgemäßen Verfahren nächstliegenden Standes der Technik, wie er durch die US-PS 3 355 504 dargestellt wird, war es nicht vorherzusehen, daß die mit diesem seit langer Zeit bekannten Verfahren verbundenen Schwierigkeiten überraschend durch den gezielten Zusatz größerer Mengen ausgewählter Lösemittel, die in unbegrenzten Mengen preiswert zur Verfügung stehen und nach abgeschlossener Umsetzung leicht abzutrennen bzw. zurückzugewinnen sind, sowie durch eine Auswahl bestimmter Verfahrensmerkmale hinsichtlich der Art und der Menge des zu verwendenden Katalysators und der einzuhaltenden Reaktionsbedingungen gelöst werden können. Nur eine derartige Kombination von Maßnahmen und die genaue Einhaltung all dieser Bedingungen führt zum Erfolg und erlaubt die Synthese von 2,6-Di-tert.butylphenol mit erheblich geringeren Anteilen an 2,4,6-Tri-tert.butylphenol. Die Anwesenheit der erfindungsgemäß zu verwendenden Lösemittel erleichtert überdies die Abführung der freiwerdenden Reaktionswärme und erlaubt die Anwesenheit eines erheblichen Isobutenüberschusses auch nach weitgehendem Umsatz des eingesetzten 2-Tert.butylphenols. Schließlich bereitet die Beseitigung des durch Hydrolyse oder auf andere Weise zu desaktivierenden Katalysators auf Grund der erfindungsgemäß zu verwendenden geringen Einsatzmengen nur einen Bruchteil der Probleme, die für die Verfahren des Standes der Technik charakteristisch sind.

Nach dem erfindungsgemäßen Verfahren läßt sich 2,6-Di-tert.butylphenol aus 2-Tert.butylphenol und Isobuten mit geringeren Anteilen an 2,4,6-Tri-tert.butylphenol herstellen, indem

a) Aluminium-tris-(2-tert.butylphenolat) als Katalysator in Mengen von 0,005 bis 5 mol-%, vorzugsweise von 0,05 bis 0,8 mol-% und besonders bevorzugt von 0,1 - 0,5 mol-%, bezogen auf eingesetztes 2-Tert.butylphenol, verwandt wird,

b) die Isobutenanlagerung bei Temperaturen von 0 °C bis 80 °C, vorzugsweise von 5° bis 50 °C und besonders bevorzugt von 10 °C bis 20 °C sowie bei Drucken von 0,1 bis 11 bar, vorzugsweise von 0,2 bis 6,0 bar und besonders bevorzugt von 0,5 bis 2,5 bar durchgeführt wird,

c) die Umsetzung in der Flüssigphase in Gegenwart von gesättigten aliphatischen und/oder cycloaliphatischen Kohlenwasserstoffen, die vorzugsweise 5 bis 10 Kohlenstoffatome enthalten, und/oder von überschüssigem flüssigem bzw. gelöstem Isobuten und/oder von $C_5$- bis $C_{12}$-Cycloalkenen und/oder von $C_5$- bis $C_{16}$-Alkenen der Formeln $R_1$-CH = $CH_2$ bzw. $R_2$-CH = CH-$R_3$ erfolgt, wobei die (Cyclo-)Alkene keine Verzweigungen an der C = C-Bindung enthalten sollten und die gesättigten Alkäne oder Cycloalkane ebenso wie Isobuten bevorzugt zu verwenden sind und

d) die genannten, unter Reaktionsbedingungen flüssigen oder gelösten Verdünnungsmittel in Mengen von 20 bis 1 000 Gewichtsteilen, vorzugsweise von 40 bis 500 Gewichtsteilen und besonders bevorzugt von 60 bis 200 Gewichtsteilen bezogen auf 100 Gewichtsteile 2-Tert.butylphenol und Isobuten in einem molaren Überschuß von 0,2 bis 10 Mol, vorzugsweise 1 bis 5 Mol, besonders bevorzugt von 1,1 bis 2,5 Mol, bezogen auf 1 Mol 2-Tert.butylphenol, zuzusetzen sind bzw. im Falle des Isobutens dieses in einem molaren Überschuß von 2 bis 10 Mol, vorzugsweise von 2,2 bis 5 Mol, pro Mol 2-Tert.butylphenol anwesend sein sollte. Es empfiehlt sich, auch bei Zusatz größerer Mengen an Verdünnungsmittel die Katalysatormenge gering zu halten.

Besonders bevorzugt als Verdünnungsmittel sind gesättigte aliphatische oder cycloaliphatische Kohlenwasserstoffe wie z. B. Hexan, Cyclohexan, Ethylcyclohexan, Isopropylcyclohexan (Hydrocumol) oder Dekalin. Bei Verwendung von (Cyclo-)Alkenen, wie z. B. Cyclohexen, cis-Cyclooocten oder Octen-1 können kleine Anteile höhersiedender phenolischer Nebenprodukte entstehen, deren destillative Abtrennung vom gewünschten 2,6-Di-tert.butylphenol bei geeigneter Wahl der C-Zahl dieser (Cyclo-)Alkene einfacher ist als die des 2,4,6-Tri-tert.butylphenols, dessen Anteil signifikant vermindert wird.

2-Tert.butylphenol und Verdünnungsmittel müssen frei von Wasser und Katalysatorgiften sein.

Unter dem Begriff Aluminium-tris-(2-tert.butylphenolat) sind auch Katalysatoren zu verstehen, bei denen einer der 2-Tert.butylphenolatreste durch andere Gruppen - wie in US-PS 3 355 504 angeführt - ersetzt sein kann.

Das nach dem erfindungsgemäßen Verfahren in besseren Ausbeuten, mit größerer Selektivität und in höherer Reinheit herstellbare 2,6-Di-tert.butylphenol ist eine gesuchte Verbindung, aus der wertvolle Folgeprodukte, vor allem auf dem Gebiet der phenolischen Antioxidantien, gewonnen werden können.

Das erfindungsgemäße Verfahren wird durch die nachfolgenden Beispiele näher erläutert.

Beispiele:

Die Alkylierungen von 2-Tert.butylphenol (Abk. 2-TBP) mit Isobuten wurden in einem Rührautoklaven bei möglichst hoher Rührerdrehzahl (≤ 1400 Upm) durchgeführt. Kühlung bzw. Temperierung des Reaktors erfolgten mit einem Hochleistungsthermostaten. Ein im Innern des Reaktors angebrachter Thermofühler erlaubte das Registrieren der Reaktionstemperatur. Veränderungen im Reaktorfüllstand konnten über eine geeignete Meßeinrichtung verfolgt werden. Proben für gaschromatographische Analysen wurden über einen Stutzen am Reaktor entnommen. Der in den Proben enthaltene Katalysator wurde durch Zusatz einiger Tropfen Wasser desaktiviert, ehe die Anteile der verschiedenen Komponenten der Reaktionsgemische gaschromatographisch bestimmt wurden. Gegebenenfalls vorhandene Beimengungen an Lösemitteln wurden bei der Auswertung der Analysen nicht berücksichtigt, d. h. die in den folgenden Tabellen enthaltenen Angaben zu den verschiedenen Beispielen geben die Zusammensetzung (in Gew.-%) von "lösungsmittelfreien" Reaktionsgemischen wieder. Dabei wurden stets folgende Abkürzungen benutzt:

2-TBP = 2-Tert.butylphenol
2,4-DTBP = 2,4-Di-tert.butylphenol
2,6-DTBP = 2,6-Di-tert.butylphenol

2,4,6-TTBP = 2,4,6-Tri-tert.butylphenol

Die angegebenen Werte der Selektivität der 2,6-Di-tert.butylphenolbildung (Abk. S) sind auf umgesetztes 2-Tert.butylphenol bezogen.

Bei sämtlichen, in den Beispielen angeführten Alkylierungen wurden jeweils 250 g (1,66 mol) 2-Tert.butylphenol in dem trockenen Rührautoklaven vorgelegt, gegebenenfalls die angegebenen Mengen der verschiedenen Lösemittel zugesetzt und anschließend die gewünschten Anteile an Katalysator durch Zugabe von Aluminiumtriethyl, gelöst in (cyclo-)aliphatischen Kohlenwasserstoffen, formiert, nachdem zuvor Reaktionsgemisch und Reaktor durch Spülen mit Inertgas von Luft (bzw. Sauerstoffresten) befreit worden waren. Nach Entfernen des freigesetzen Ethans wurde der Reaktor verschlossen und sein Inhalt temperiert. Bei der gewünschten Reaktionstemperatur wurde anschließend unter kräftiger Durchmischung Isobuten in der vorgesehenen Menge zudosiert. Die isotherm durchgeführten Alkylierungen wurden im allgemeinen beendet, sobald hohe 2,6-Di-tert.butylphenolanteile erreicht waren bzw. keine nennenswerte weitere Abnahme des 2-Tert.butylphenolgehalts zu beobachten war.

Bedingt durch das in nur kleinen Mengen verwandte Katalysatorsystem und dessen Empfindlichkeit gegen bestimmte Verunreinigungen sind Vergleiche nur innerhalb einer Serie von Versuchen ratsam, bei der gleiche Chargen an Edukt, Lösemittel und Aluminiumtriethyl zum Einsatz kamen.

Aus Gründen der Übersicht ist in den Tabellen stets der verwandte Überschuß an Isobuten angegeben, d. h. die Differenz zwischen zugesetzter Gesamtmenge an Isobuten und der bei vollständigem Umsatz der eingesetzten 2-Tert.butylphenol-menge zu 2,6-DTBP erforderlichen Isobutenmenge (1,66 mol). Bei den meisten Versuchen nach dem erfindungsgemäßen Verfahren wurde mit größerem Isobutenüberschuß gearbeitet als bei den Vergleichsversuchen nach dem Stand der Technik. Bei den in bar angegebenen Drucken handelt es sich um die im System eingestellten Gesamtdrucke (Absolutdrucke).

Beispiele 1 bis 8 (nicht erfindungsgemäß)

In der zuvor beschriebenen Weise wurden 250 g (1,66 mol) 2-Tert.butylphenol bei 1,5 bar mit einem begrenzten Überschuß an Isobuten in Gegenwart unterschiedlicher Mengen Aluminium-tris-(2-tert.butylphenolat) bei 10 °C bzw. 30 °C umgesetzt. Tabelle I enthält die nach unterschiedlichen Reaktionszeiten beobachteten Anteile an Alkylphenolen in den Reaktionsgemischen.

In allen Fällen wird ein hoher Anteil an 2,4,6-Tri-tert.butylphenol beobachtet, sobald das eingesetzte 2-Tert.butylphenol weitgehend verbraucht und in unterschiedlichem Maße in 2,6-Di-tert.butylphenol umgewandelt ist (vgl. z. B. Beispiel 5 mit Beispiel 9, Beispiel 6 mit Beispiel 10, Beispiel 8 mit Beispiel 12 usw.).

Beispiele 9 bis 12 (vgl. Tabelle I)

In der bei den Beispielen 1 bis 8 angewandten Arbeitsweise wurde 2-Tert.butylphenol bei 10 °C bzw. 30 °C in Gegenwart geringer Katalysatormengen bei einem Druck von 2,5 bis 2,8 bar mit einem deutlichen Überschuß an Isobuten alkyliert. Dadurch konnte der Anteil des unerwünschten 2,4,6-Tri-tert.butylphenols gegenüber dem Stand der Technik auch bei hohem 2-Tert.butylphenol-umsatz auf einem niedrigeren Niveau gehalten werden.

Beispiele 13 bis 30 (vgl. Tabelle II)

Nach der zuvor beschriebenen Verfahrensweise wurden 250 g (1,66 mol) 2-Tert.butylphenol in Gegenwart der angegebenen kleinen Mengen an Aluminium-tris-(2-tert.butylphenolat) bei 10 °C bzw. 30 °C mit einem Überschuß an Isobuten bei einem Druck von 1,5 bis 2,8 bar alkyliert. Dabei wurden unterschiedliche Mengen verschiedener Lösemittel zugesetzt.

Man erkennt, daß in Abwesenheit der erfindungsgemäß zuzusetzenden (cyclo-)aliphatischen Lösemittel höhere Anteile an 2,4,6-Tri-tert.butylphenol(und entsprechend niedrigere Selektivitäten bei der 2,6-Di-tert.butylphenol-bildung) unter vergleichbaren Reaktionsbedingungen beobachtet werden. Mit Toluol (als Vertreter aromatischer Lösemittel) konnte eine nennenswerte Absenkung des 2,4,6-Tri-tert.butylphenolgehalts nicht erreicht werden.

Bespiele 31 bis 59 (vgl. Tabelle III und IV)

In der bei den Beispielen 1 bis 30 angewandten Arbeitsweise wurden 250 g (1,66 mol) 2-Tert.butylphenol in Gegenwart von 1,9 bis 8,5 mmol Aluminium-tris-(2-tert.butylphenolat) mit einem Überschuß an Isobuten bei 1,4 bis 1,5 bar alkyliert. Die Ergebnisse zeigen, daß durch Verwendung steigender Mengen der erfindungsgemäßen Lösemittel verbesserte Selektivitäten bei der 2,6-Di-tert.butylphenolbildung zu erzielen sind und daß unter derartigen Bedingungen selbst mit einem hohen Isobutenüberschuß auch nach erheblich verlängerten Reaktionszeiten nur ein geringer Anstieg des 2,4,6-Tri-tert.butylphenol-anteilszu beobachten ist.

In gleicher Weise wirken sich in Anwesenheit der erfindungsgemäß einzusetzenden Lösemittel die Verminderung der Katalysatormenge bzw. der Reaktionstemperatur vorteilhaft aus. - Mit Toluol bzw. m-Xylol sind vergleichbare Verbesserungen unter identischen Reaktionsbedingungen nicht zu erzielen, wohl aber mit den Lösemitteln Ethylcyclohexan, Isopropylcyclohexan oder Dekalin, deren Verwendung nach dem erfindungsgemäßen Verfahren ebenfalls von Vorteil sein sollte.

Beispiele 60 bis 63 (vgl. Tabell V)

In der bei den Beispielen 1 bis 59 angewandten Arbeitsweise wurden 250 g (1,66 mol) 2-Tert.butylphenol mit einem Überschuß an Isobuten bei einem Druck von 1,5 bar und einer Reaktionstemperatur von 10 ˚ C in Gegenwart von 5,7 mol Aluminium-tris-(2-tert.butylphenolat) umgesetzt. In Anwesenheit von jeweils 200 ml verschiedener, erfindungsgemäß zuzusetzender Olefine konnte der Anteil des unerwünschten 2,4,6-Tri-tert.butylphenols deutlich gesenkt werden, ohne daß ein vergleichbarer Anteil unerwünschter, schwierig destillativ abtrennbarer Verunreinigungen entstand, selbst wenn die Reaktionszeit erheblich über den sonst üblichen bzw. zur Erzielung eines hohen 2-Tert.butylphenol-umsatzes erforderlichen Zeitraum hinaus verlängert wurde.

EP 0 438 641 A2

Tabelle I: Herstellung von 2,6-Di-tert.butylphenol aus 2-Tert.butylphenol

| Beispiel-Nr. | Katal.-menge (mmol) | Isobuten-überschuß (mol) | Druck (bar) | Reaktions-temperatur (°C) | Reaktions-zeit (Stdn.) | Zusammensetzung Reaktionsprodukt (Gew.-%) | | | | Selektivi-tät S |
|---|---|---|---|---|---|---|---|---|---|---|
| | | | | | | 2-TBP | 2,6-DTBP | 2,4-DTBP | 2,4,6-TTBP | |
| 1* | 45 | 1,5 | 1,4 | 10-30 | 0,7 | 3,7 | 89,0 | 0,3 | 6,3 | 94,4 |
| 2* | 22,5 " | 1,6 " | 1,5 " | 10 " | 0,7 | 9,4 | 85,8 | 0,3 | 4,0 | 96,1 |
| | | | | | 1,0 | 1,4 | 93,3 | 0,1 | 4,7 | 96,1 |
| 3* | 11,3 " " " | 1,6 " " " | 1,5 " " " | 10 " " " | 0,7 | 24,1 | 71,6 | 0,7 | 3,2 | 95,7 |
| | | | | | 1,0 | 5,2 | 89,8 | 0,1 | 4,3 | 96,3 |
| | | | | | 1,5 | 1,0 | 94,0 | 0,1 | 4,4 | 96,4 |
| | | | | | 3,0 | 0,8 | 92,5 | 0,1 | 6,0 | 95,0 |
| 4* | 8,5 " " | 1,6 " " | 1,5 " " | 10 " " | 0,7 | 33,0 | 63,3 | 0,8 | 2,5 | 95,8 |
| | | | | | 1,0 | 12,5 | 83,5 | 0,3 | 3,4 | 96,6 |
| | | | | | 1,5 | 0,9 | 94,6 | 0,05 | 4,2 | 96,5 |
| 5* | 8,5 " | 1,6 " | 1,5 " | 10 " | 1,0 | 16,3 | 79,3 | 0,4 | 3,5 | 96,1 |
| | | | | | 3,0 | 1,2 | 93,9 | 0,07 | 4,3 | 96,4 |
| 6* | " " | 1,5 " | 1,7 " | 30 " | 1,0 | 11,9 | 80,5 | 0,5 | 6,3 | 93,6 |
| | | | | | 3,0 | 1,6 | 90,5 | 0,09 | 7,1 | 94,1 |
| 7* | 5,7 " " | 1,6 " " | 1,5 " " | 10 " " | 1,0 | 38,9 | 57,9 | 0,9 | 1,9 | 96,0 |
| | | | | | 3,0 | 8,1 | 87,7 | 0,2 | 3,6 | 96,7 |
| | | | | | 5,0 | 4,2 | 91,7 | 0,08 | 3,7 | 96,8 |
| 8* | " " " | 1,5 " " | " " " | 30 " " | 1,0 | 27,9 | 65,5 | 1,3 | 4,6 | 93,0 |
| | | | | | 3,0 | 6,7 | 85,9 | 0,3 | 6,5 | 94,1 |
| | | | | | 5,0 | 1,6 | 91,2 | 0,07 | 6,5 | 94,5 |

8

Tabelle I: - Fortsetzung -

| Beispiel-Nr. | Katal.-menge (mmol) | Isobuten-überschuß (mol) | Druck (bar) | Reaktions-temperatur (°C) | Reaktions-zeit (Stdn.) | Zusammensetzung Reaktionsprodukt (Gew.-%) | | | | Selektivität S |
|---|---|---|---|---|---|---|---|---|---|---|
| | | | | | | 2-TBP | 2,6-DTBP | 2,4-DTBP | 2,4,6-TTBP | |
| 9 | 8,5 " | 3,0 " | 2,6 " | 10 " | 1,0 3,0 | 8,9 2,5 | 87,5 93,7 | 0,14 0,07 | 3,0 3,4 | 97,2 97,2 |
| 10 | " " | 2,8 " | 2,8 " | 30 " | 1,0 3,0 | 2,0 0,5 | 92,2 93,6 | 0,08 0,04 | 5,0 5,2 | 95,8 95,7 |
| 11 | 5,7 " | 2,9 " | 2,5 " | 10 " | 1,0 3,0 | 60,7 23,1 | 38,2 75,2 | 0,5 0,3 | 0,5 1,2 | 97,7 98,3 |
| 12 | " " " | 2,7 " " | 2,7 " " | 30 " " | 1,0 3,0 5,0 | 19,8 3,3 1,7 | 75,7 91,5 93,0 | 0,5 0,09 0,06 | 3,5 4,6 4,7 | 95,9 96,1 96,1 |

Abkürzungen: 2-TBP      :  2-Tert.butylphenol
2,6-DTBP  :  2,6-Di-tert.butylphenol
2,4-DTBP  :  2,4-Di-tert.butylphenol
2,4,6-TTBP:  2,4,6-Tri-tert.butylphenol
*         :  nicht erfindungsgemäß

EP 0 438 641 A2

Tabelle II: Herstellung von 2,6-Di-tert.butylphenol aus 2-Tert.butylphenol

| Beisp. Nr. | Katal.- menge (mmol) | Isobuten- überschuß (mol) | Lösungsmittel Art | Menge (ml) | Druck (bar) | Reakt. temp. (°C) | Reakt.- zeit (Stdn.) | Zusammensetzung Reaktionsprodukt (Gew.-%) | | | | Selek- tivität S |
|---|---|---|---|---|---|---|---|---|---|---|---|---|
| | | | | | | | | 2-TBP | 2,6-DTBP | 2,4-DTBP | 2,4,6-TTBP | |
| 13* | 8,5 " | 2,7 " | - | | 2,8 " | 30 " | 1,0 3,0 | 2,6 0,6 | 91,5 93,6 | 0,1 0,04 | 5,1 5,3 | 95,6 95,7 |
| 14* | " " " | 3,2 " " | Toluol " " | 100 " " | 2,6 " " | " " " | 1,0 3,0 5,0 | 5,5 1,1 0,7 | 88,8 92,9 93,2 | 0,2 0,1 0,07 | 4,9 5,3 5,4 | 95,5 95,6 95,5 |
| 15 | " " " | 3,4 " " | n-Hexan " " | 100 " " | 2,6 " " | " " " | 1,0 3,0 5,0 | 8,6 1,4 0,9 | 87,8 94,8 95,2 | 0,2 0,05 0,05 | 2,9 3,3 3,4 | 97,2 97,3 97,2 |
| 16 | " " " | 3,3 " " | Cyclohexan " " | 100 " " | 2,7 " " | " " " | 1,0 3,0 5,0 | 5,3 1,0 0,8 | 91,3 95,3 95,6 | 0,12 0,06 0,05 | 2,9 3,1 3,2 | 97,4 97,4 97,3 |
| 17 | " " " | 4,6 " " | Cyclohexan " " | 200 " " | 2,6 " " | " " " | 1,0 3,0 5,0 | 11,7 2,4 1,0 | 86,0 95,0 96,4 | 0,2 0,07 0,04 | 1,9 2,2 2,2 | 98,0 98,1 98,1 |
| 18* | 5,7 " " | 2,8 " " | - | | 2,8 " " | 30 " " | 1,0 3,0 5,0 | 18,9 3,2 1,6 | 76,6 91,7 93,9 | 0,5 0,09 0,07 | 3,5 4,5 4,5 | 95,8 96,2 96,2 |
| 19 | " " " | 3,3 " " | Cyclohexan " " | 50 " " | 2,7 " " | " " " | 1,0 3,0 5,0 | 34,6 10,4 5,9 | 63,0 86,4 90,8 | 0,6 0,2 0,12 | 1,5 2,6 2,8 | 97,2 97,5 97,5 |
| 20 | " " " | 4,3 " " | Cyclohexan " " | 100 " " | 2,7 " " | " " " | 1,0 3,0 5,0 | 50,9 22,0 14,7 | 47,6 75,8 83,0 | 0,6 0,3 0,2 | 0,7 1,6 1,8 | 97,6 98,0 98,1 |

EP 0 438 641 A2

Tabelle II: - Fortsetzung -

| Beisp. Nr. | Katal.-menge (mmol) | Isobuten-überschuß (mol) | Lösungsmittel Art | Menge (ml) | Druck (bar) | Reakt.-temp. (°C) | Reakt.-zeit (Stdn.) | 2-TBP | 2,6-DTBP | 2,4-DTBP | 2,4,6-TTBP | Selektivität S |
|---|---|---|---|---|---|---|---|---|---|---|---|---|
| 21* | 8,5 | 1,6 | - | | 1,5 | 10 | 1,0 | 23,1 | 72,8 | 0,6 | 3,0 | 96,1 |
| | " | " | | | " | " | 3,0 | 1,7 | 93,7 | 0,1 | 4,1 | 96,6 |
| | " | " | | | " | " | 5,0 | 0,9 | 94,1 | 0,1 | 4,4 | 96,4 |
| 22 | " | 1,8 | Cyclohexan | 50 | 1,5 | " | 1,0 | 21,1 | 76,0 | 0,4 | 2,2 | 97,3 |
| | " | " | " | " | | | 3,0 | 1,8 | 94,7 | 0,1 | 3,0 | 97,5 |
| 23 | " | 1,8 | Cyclohexan | 100 | 1,5 | " | 1,0 | 18,6 | 79,1 | 0,3 | 1,7 | 98,0 |
| | " | " | " | " | " | " | 3,0 | 1,6 | 95,7 | 0,09 | 2,3 | 98,1 |
| | " | " | " | " | " | " | 5,0 | 1,0 | 96,3 | 0,09 | 2,4 | 98,0 |
| 24 | " | 2,1 | Cyclohexan | 200 | 1,5 | " | 1,0 | 25,5 | 73,0 | 0,3 | 1,0 | 98,5 |
| | " | " | " | " | " | " | 3,0 | 3,2 | 94,6 | 0,08 | 1,6 | 98,6 |
| | " | " | " | " | " | " | 5,0 | 1,3 | 96,7 | 0,06 | 1,6 | 98,7 |
| 25* | 8,5 | 1,1 | - | " | 1,7 | 30 | 1,0 | 14,7 | 78,2 | 0,6 | 5,8 | 93,8 |
| | " | " | " | " | " | " | 3,0 | 2,7 | 89,4 | 0,12 | 7,1 | 94,0 |
| | " | " | " | " | " | " | 5,0 | 1,2 | 90,9 | 0,08 | 7,1 | 94,1 |
| 26 | " | 1,1 | Cyclohexan | 100 | 1,5 | " | 1,0 | 24,7 | 71,6 | 0,6 | 2,5 | 96,4 |
| | " | " | " | " | | | 3,0 | 6,1 | 87,8 | 0,2 | 3,5 | 96,7 |
| | " | " | | | | | 5,0 | 3,5 | 92,5 | 0,1 | 3,6 | 96,9 |
| 27 | " | 1,7 | Cyclohexan | 200 | 1,5 | " | 1,0 | 44,9 | 53,3 | 0,6 | 0,9 | 97,6 |
| | " | " | " | " | " | " | 3,0 | 17,3 | 80,0 | 0,3 | 2,0 | 97,6 |
| | " | " | " | " | " | " | 5,0 | 10,3 | 86,9 | 0,2 | 2,2 | 97,8 |
| 28 | 5,7 | 3,9 | Cyclohexan | 200 | 1,5 | 10 | 1,0 | 46,6 | 53,4 | 0,3 | 0,5 | 98,7 |
| | " | " | " | " | " | " | 3,0 | 9,5 | 89,0 | 0,1 | 1,2 | 98,8 |
| | " | " | " | " | " | " | 5,0 | 3,2 | 95,2 | 0,07 | 1,4 | 98,8 |
| | " | " | " | " | " | " | 22,0 | 0,7 | 97,6 | 0,07 | 1,5 | 98,7 |

EP 0 438 641 A2

Tabelle II: - Fortsetzung -

| Beisp. Nr. | Katal.- menge (mmol) | Isobuten- überschuß (mol) | Lösungsmittel Art | Menge (ml) | Druck (bar) | Reakt. temp. (°C) | Reakt.- zeit (Stdn.) | Zusammensetzung Reaktionsprodukt (Gew.-%) | | | | Selek- tivität S |
|---|---|---|---|---|---|---|---|---|---|---|---|---|
| | | | | | | | | 2-TBP | 2,6-DTBP | 2,4-DTBP | 2,4,6-TTBP | |
| 29* | 8,5 " " | 1,1 " " | - | | 1,5 " " | 30 " " | 1,0 3,0 5,0 | 23,6 5,1 1,7 | 69,8 87,3 90,3 | 1,1 0,3 0,14 | 4,9 6,5 7,1 | 93,4 94,2 94,0 |
| 30* | " " " | 1,3 " " | Toluol " " | 100 " " | 1,5 " " | " " " | 1,0 3,0 5,0 | 24,5 4,1 1,7 | 68,8 88,2 90,5 | 1,2 0,3 0,2 | 4,6 6,8 6,9 | 93,5 94,0 94,1 |

* = nicht erfindungsgemäß

EP 0 438 641 A2

EP 0 438 641 A2

Tabelle III: Herstellung von 2,6-Di-tert.butylphenol aus 2-Tert.butylphenol

| Beisp. Nr. | Katal.-menge (mmol) | Isobuten-überschuß (mol) | Lösungsmittel Art | Menge (ml) | Druck (bar) | Reakt.-temp. (°C) | Reakt.-zeit (Stdn.) | 2-TBP | 2,6-DTBP | 2,4-DTBP | 2,4,6-TTBP | Selek-tivität S |
|---|---|---|---|---|---|---|---|---|---|---|---|---|
| | | | | | | | | Zusammensetzung Reaktionsprodukt (Gew.-%) | | | | |
| 31* | 8,5 " " | 1,0 " " | - | | 1,5 " " | 30 " " | 1,0 3,0 5,0 | 29,2 5,9 2,4 | 64,6 86,6 90,0 | 1,2 0,3 0,15 | 4,4 6,5 6,7 | 93,3 94,1 94,3 |
| 32* | " " " | 1,3 " " | m-Xylol " " | 100 | 1,5 " " | " " " | 1,0 3,0 5,0 | 29,3 5,1 2,0 | 65,4 87,9 90,8 | 1,2 0,3 0,14 | 3,6 6,1 6,5 | 94,2 94,5 94,5 |
| 33* | 8,5 " " | 1,8 " " | m-Xylol " " | 100 | 1,5 " " | 10 " " | 1,0 3,0 5,0 | 30,3 2,3 0,9 | 66,3 93,4 94,6 | 0,7 0,1 0,08 | 2,3 3,8 4,0 | 96,4 96,8 96,7 |
| 34 | " " " | " " " | Hexan " " | " " " | " " " | " " " | 1,0 3,0 5,0 | 20,7 1,6 0,8 | 76,5 95,3 96,0 | 0,4 0,07 0,07 | 2,0 2,8 2,8 | 97,5 97,7 97,7 |
| 35 | " " " | 1,9 " " | Hexan " " | 200 | 1,5 " " | 10 " " | 1,0 3,0 5,0 | 29,8 4,5 1,7 | 68,3 93,0 95,7 | 0,4 0,08 0,06 | 1,3 2,1 2,2 | 98,0 98,2 98,2 |
| 36* | 8,5 " " | 1,3 " " | - | - | 1,5 " " | 30 " " | 1,0 3,0 5,0 | 5,6 1,7 1,3 | 85,7 88,5 88,9 | 0,3 0,4 0,4 | 7,6 8,5 8,6 | 93,2 92,6 92,6 |

Tabelle III: - Fortsetzung -

| Beisp. Nr. | Katal.-menge (mmol) | Isobuten-überschuß (mol) | Lösungsmittel Art | Menge (ml) | Druck (bar) | Reakt.-temp. (°C) | Reakt.-zeit (Stdn.) | Zusammensetzung Reaktionsprodukt (Gew.-%) | | | | Selek-tivität S |
|---|---|---|---|---|---|---|---|---|---|---|---|---|
| | | | | | | | | 2-TBP | 2,6-DTBP | 2,4-DTBP | 2,4,6-TTBP | |
| 37 | 8,5 " " | 2,3 " " | Cyclohexan " " | 200 " " | 1,5 " " | 10 " " | 1,0 3,0 5,0 | 5,7 2,0 2,0 | 92,4 95,6 95,5 | 0,1 0,14 0,15 | 1,6 2,0 2,1 | 98,5 98,2 98,2 |
| 38 | 5,7 " " | 2,4 " " | " " " | " " " | " " " | " " " | 1,0 3,0 5,0 | 15,9 1,5 1,5 | 82,5 96,3 96,3 | 0,16 0,1 0,1 | 1,2 1,8 1,8 | 98,7 98,5 98,5 |
| 39 | 3,8 " " " | 1,8 " " " | Cyclohexan " " " | 200 " " " | 1,5 " " " | 10 " " " | 1,0 3,0 5,0 11,0 | 39,8 8,0 3,0 0,8 | 58,9 90,1 94,9 97,0 | 0,3 0,13 0,06 0,05 | 0,7 1,5 1,7 1,9 | 98,6 98,6 98,6 98,4 |
| 40 | 2,8 " " " | " " " " | " " " " | " " " " | " " " " | " " " " | 1,0 5,0 12,0 22,0 | 63,6 10,5 2,5 0,8 | 35,6 87,8 95,6 97,3 | 0,3 0,1 0,04 0,03 | 0,3 1,3 1,6 1,6 | 98,5 98,7 98,7 98,7 |
| 41 | 1,9 " " " " | 1,7 " " " " | " " " " " | " " " " " | " " " " " | " " " " " | 1,0 5,0 12,0 21,0 49,0 | 81,1 30,8 8,4 3,6 1,0 | 18,5 67,9 89,9 94,6 97,1 | 0,2 0,3 0,09 0,05 0,02 | 0,1 0,9 1,4 1,5 1,6 | 98,5 98,5 98,7 98,7 98,7 |
| 42* | 5,7 " " | 2,0 " " | Xylol " " | " " " | " " " | " " " | 1,0 3,0 5,0 | 15,1 1,1 0,9 | 81,0 94,3 93,9 | 0,4 0,1 0,1 | 3,1 4,1 4,7 | 96,6 96,6 96,1 |

Tabelle III: - Fortsetzung -

| Beisp. Nr. | Katal.-menge (mmol) | Isobuten-überschuß (mol) | Lösungsmittel Art | Menge (ml) | Druck (bar) | Reakt. temp. (°C) | Reakt.-zeit (Stdn.) | Zusammensetzung Reaktionsprodukt (Gew.-%) 2-TBP | 2,6-DTBP | 2,4-DTBP | 2,4,6-TTBP | Selek-tivität S |
|---|---|---|---|---|---|---|---|---|---|---|---|---|
| 43* | 5,7 | 1,0 | - | | 1,5 | 50 | 1,0 | 20,4 | 68,4 | 1,5 | 8,6 | 89,2 |
| | " | " | | | " | " | 3,0 | 3,7 | 83,7 | 0,5 | 11,0 | 90,1 |
| | " | " | | | | " | 5,0 | 2,3 | 85,1 | 0,3 | 11,2 | 90,3 |
| 44 | 5,7 | 1,2 | Hexan | 400 | " | " | 1,0 | 36,3 | 61,0 | 0,7 | 1,6 | 96,9 |
| | " | " | " | " | " | " | 3,0 | 12,0 | 84,4 | 0,3 | 2,9 | 97,0 |
| | " | " | " | " | " | " | 5,0 | 7,2 | 88,9 | 0,2 | 3,2 | 97,0 |
| | " | " | " | " | " | " | 22,0 | 2,2 | 93,0 | 0,1 | 3,6 | 97,0 |
| 45 | " | 2,1 | " | " | " | 10 | 1,0 | 32,1 | 66,4 | 0,3 | 0,8 | 98,6 |
| | " | " | " | " | " | " | 3,0 | 9,4 | 88,9 | 0,1 | 1,3 | 98,7 |
| | " | " | " | " | " | " | 5,0 | 4,0 | 94,2 | 0,07 | 1,5 | 98,7 |
| | " | " | " | " | " | " | 22,0 | 1,2 | 96,3 | 0,20 | 2,0 | 98,2 |
| 46 | 8,5 | 4,8 | Hexan | 800 | 1,5 | 10 | 1,0 | 25,0 | 73,9 | 0,2 | 0,7 | 99,0 |
| | " | " | " | " | " | " | 3,0 | 6,3 | 92,3 | 0,09 | 1,1 | 99,0 |
| | " | " | " | " | " | " | 5,0 | 3,0 | 95,3 | 0,09 | 1,3 | 98,8 |
| | " | " | " | " | " | " | 22,0 | 1,9 | 96,0 | 0,1 | 1,5 | 98,6 |

* = nicht erfindungsgemäß

EP 0 438 641 A2

Tabelle IV: Herstellung von 2,6-Di-tert.butylphenol aus 2-Tert.butylphenol

| Beisp. Nr. | Katal.-menge (mmol) | Isobuten-überschuß (mol) | Lösungsmittel Art | Menge (ml) | Druck (bar) | Reakt.-temp. (°C) | Reakt.-zeit (Stdn.) | Zusammensetzung Reaktionsprodukt (Gew.-%) | | | | Selek-tivität S |
|---|---|---|---|---|---|---|---|---|---|---|---|---|
| | | | | | | | | 2-TBP | 2,6-DTBP | 2,4-DTBP | 2,4,6-TTBP | |
| 47* | 5,7 " " | 1,3 " " | - | | 1,4 " " | 10 " " | 1,0 3,0 5,0 | 21,1 1,1 0,8 | 73,8 92,9 93,4 | 0,7 0,2 0,13 | 3,8 5,3 5,1 | 95,2 95,5 95,8 |
| 48 | 5,7 " " " | 1,6 " " " | Hexan " " " | 1250 " " " | 1,5 " " " | " " " " | 1,0 3,0 5,0 22,0 | 68,7 29,0 15,3 2,0 | 39,4 70,0 83,5 96,3 | 0,2 0,2 0,14 0,07 | 0,2 0,6 0,9 1,3 | 99,1 99,0 99,0 98,9 |
| 49 | 8,5 " " " | 1,8 " " " | " " " " | " " " " | " " " " | " " " " | 1,0 3,0 5,0 22,0 | 49,4 18,0 9,0 2,0 | 49,9 80,8 89,7 96,3 | 0,2 0,15 0,1 0,09 | 0,3 0,8 1,1 1,4 | 99,1 99,0 98,9 98,8 |
| 50 | 8,9 " " " | 1,6 " " " | " " " " | 830 " " " | 1,5 " " " | 50 " " " | 1,0 3,0 5,0 22,0 | 32,7 9,1 4,3 2,4 | 65,5 88,2 93,0 94,2 | 0,4 0,3 0,2 0,3 | 1,0 2,1 2,2 2,7 | 98,2 97,8 98,0 97,5 |
| 51 | 8,9 " " " | 2,3 " " " | " " " " | " " " " | 1,5 " " " | 30 " " " | 1,0 3,0 5,0 7,0 | 23,4 5,0 2,5 1,8 | 75,3 93,1 95,5 96,1 | 0,2 0,1 0,1 0,1 | 0,9 1,5 1,6 1,7 | 98,8 98,6 98,6 98,5 |
| 52 | " " " " | 2,6 " " " | " " " " | " " " " | " " " " | 10 " " " | 1,0 3,0 5,0 21,0 | 37,6 12,8 6,5 2,0 | 61,5 85,9 92,2 96,3 | 0,25 0,14 0,1 0,14 | 0,5 0,9 1,1 1,3 | 99,0 99,0 99,0 98,8 |

EP 0 438 641 A2

Tabelle IV: - Fortsetzung -

| Beisp. Nr. | Katal.-menge (mmol) | Isobuten-überschuß (mol) | Lösungsmittel Art | Menge (ml) | Druck (bar) | Reakt.-temp. (°C) | Reakt.-zeit (Stdn.) | Zusammensetzung Reaktionsprodukt (Gew.-%) | | | | Selek-tivität S |
|---|---|---|---|---|---|---|---|---|---|---|---|---|
| | | | | | | | | 2-TBP | 2,6-DTBP | 2,4-DTBP | 2,4,6-TTBP | |
| 53* | 8,5 " " | 2,0 " " | - | | 1,5 " " | 10 " " | 1,0 3,0 5,0 | 2,0 1,3 1,3 | 92,1 91,8 91,0 | 0,05 0,07 0,09 | 5,2 6,2 6,9 | 95,7 94,9 94,3 |
| 54* | 5,7 " " " | 1,7 " " " | - | | 1,5 " " " | 10 " " " | 1,0 3,0 5,0 21,0 | 17,7 1,2 0,8 0,7 | 78,0 93,3 93,8 92,8 | 0,15 0,03 0,03 0,07 | 3,7 4,6 4,8 5,3 | 96,2 96,2 96,1 95,6 |
| 55 | 5,7 " " " " | 2,2 " " " " | Ethylcy-clohexan " " " | 200 " " " " | " " " " " | " " " " " | 1,0 3,0 5,0 7,0 69,0 | 35,4 7,6 3,2 1,7 1,0 | 63,2 90,3 94,5 95,9 95,7 | 0,3 0,04 0,05 0,03 0,08 | 0,9 1,8 1,9 2,0 2,9 | 98,4 98,4 98,4 98,4 97,6 |
| 56 | " " " " | 1,8 " " " | Isopropyl-cyclohexan " " | " " " " | " " " " | " " " " | 1,0 3,0 5,0 21,0 | 32,8 6,8 2,7 0,9 | 65,5 90,9 95,0 96,2 | 0,3 0,04 0,03 0,07 | 1,1 1,8 2,0 2,5 | 98,3 98,4 98,3 97,9 |
| 57 | " " " " | 1,8 " " " | Dekalin " " " | " " " " | " " " " | " " " " | 1,0 3,0 5,0 21,0 | 32,5 6,7 2,6 0,9 | 65,8 91,1 95,1 96,1 | 0,3 0,04 0,03 0,07 | 1,1 1,8 2,0 2,4 | 98,3 98,4 98,3 98,0 |

EP 0 438 641 A2

EP 0 438 641 A2

Tabelle IV: - Fortsetzung -

| Beisp. Nr. | Katal.-menge (mmol) | Isobuten-überschuß (mol) | Lösungsmittel Art | Menge (ml) | Druck (bar) | Reakt.-temp. (°C) | Reakt.-zeit (Stdn.) | Zusammensetzung Reaktionsprodukt (Gew.-%) 2-TBP | 2,6-DTBP | 2,4-DTBP | 2,4,6-TTBP | Selektivität S |
|---|---|---|---|---|---|---|---|---|---|---|---|---|
| 58* | 5,7 | 1,9 | Toluol | 200 | 1,5 | 10 | 1,0 | 29,9 | 66,5 | 0,7 | 2,5 | 96,1 |
| | " | " | " | " | " | " | 3,0 | 2,8 | 92,0 | 0,05 | 4,1 | 96,6 |
| | " | " | " | " | " | " | 5,0 | 0,9 | 93,8 | 0,04 | 4,8 | 96,1 |
| | " | " | " | " | " | " | 21,0 | 0,8 | 93,8 | 0,05 | 4,8 | 96,1 |
| 59* | " | 2,6 | m-Xylol | " | " | " | 1,0 | 20,7 | 76,0 | 0,2 | 2,6 | 97,1 |
| | " | " | " | " | " | " | 3,0 | 0,9 | 94,6 | 0,04 | 4,0 | 96,7 |
| | " | " | " | " | " | " | 5,0 | 0,8 | 94,5 | 0,05 | 4,2 | 96,6 |

* = nicht erfindungsgemäß

Tabelle V: Herstellung von 2,6-Di-tert.butylphenol aus 2-Tert.butylphenol

| Beisp. Nr. | Katal.-menge (mmol) | Isobuten-überschuß (mol) | Lösungsmittel Art | Menge (ml) | Druck (bar) | Reakt.-temp. (°C) | Reakt.-zeit (Stdn.) | Zusammensetzung Reaktionsprodukt (Gew.-%) | | | | Hoch-sieder Gew.-% |
|---|---|---|---|---|---|---|---|---|---|---|---|---|
| | | | | | | | | 2-TBP | 2,6-DTBP | 2,4-DTBP | 2,4,6-TTBP | |
| 60* | 5,7 " " " | 1,7 " " " | - | | 1,5 " " " | 10 " " " | 1,0 3,0 5,0 50,0 70,0 | 20,7 1,5 0,8 0,8 0,7 | 74,9 93,1 93,4 91,2 90,6 | 0,2 0,07 0,05 0,09 0,07 | 3,7 4,8 5,1 7,3 7,9 | |
| 61 | 5,7 " " " | 2,0 " " " | Cyclohexen | 200 | 1,5 " " | 10 " " | 1,0 3,0 5,0 22,0 | 48,3 15,6 6,7 1,0 | 50,1 82,1 90,5 95,8 | 0,4 0,09 0,05 0,03 | 0,7 1,7 2,2 2,5 | - - - - |
| 62 | 5,7 " " " | 2,0 " " " | Cycloocten " " " | 200 " " " | " " " " | " " " " | 1,0 3,0 5,0 22,0 | 32,2 6,9 2,5 1,1 | 63,1 87,9 92,4 93,5 | 0,4 0,13 0,03 0,07 | 1,1 2,0 2,2 2,5 | 2,7 2,7 2,6 2,5 |
| 63 | " " " " | 1,9 " " " | Octen-(1) " " " | " " " " | " " " " | " " " " | 1,0 3,0 5,0 22,0 | 32,8 6,8 2,6 0,9 | 62,8 88,3 92,4 93,7 | 0,4 0,05 0,03 0,06 | 1,2 2,2 2,4 2,7 | 2,3 2,3 2,2 2,1 |

## Patentansprüche

1. Verfahren zur Herstellung von 2,6-Di-tert.butylphenol durch Umsetzung von 2-Tert.butylphenol mit Isobuten in der Flüssigphase in Gegenwart von Aluminium-tris-(2-tert.butylphenolat) als Katalysator, dadurch gekennzeichnet, daß man die Umsetzung in Gegenwart von gesättigten aliphatischen und/oder cycloaliphatischen Kohlenwasserstoffen und/oder von überschüssigem flüssigem bzw. gelöstem Isobuten und/oder von

$C_5$- bis $C_{16}$-Alkenen der Formeln $R_1$-CH $=$ CH$_2$ oder $R_2$-CH $=$ CH-$R_3$, in denen $R_2$ und $R_3$ auch unter Bildung eines $C_5$- bis $C_{12}$-Cycloalkens verknüpft sein können, als Verdünnungsmittel bei Temperaturen von 0 °C bis 80 °C, bei Drucken von 0,1 bis 11 bar und mit Katalysatormengen von 0,005 bis 5 mol-%, bezogen auf eingesetztes 2-Tert.butylphenol, durchführt.

2. Verfahren nach Anspruch 1,
dadurch gekennzeichnet,
daß die gesättigten aliphatischen und/oder cycloaliphatischen Kohlenwasserstoffe und/oder die $C_5$- bis $C_{16}$- Alkene bzw. die $C_5$- bis $C_{12}$-Cycloalkene in Mengen von 20 bis 1 000 Gewichtsteilen, bezogen auf 100 Gewichtsteile 2-Tert.butylphenol, und Isobuten in einem molaren Überschuß von 0,2 bis 10 Mol, bezogen auf 1 Mol 2-Tert.butylphenol, eingesetzt werden.

3. Verfahren nach Anspruch 1,
dadurch gekennzeichnet,
daß die Reaktion in Gegenwart eines Isobutenüberschusses von 2 bis 10 Mol, bezogen auf 1 Mol 2-Tert.butylphenol, durchgeführt wird.

4. Verfahren nach Anspruch 1,
dadurch gekennzeichnet,
daß als Verdünnungsmittel Hexan, Cyclohexan, Ethylcyclohexan, Isopropylcyclohexan oder Dekalin eingesetzt werden.

5. Verfahren nach den Ansprüchen 1 und 2,
dadurch gekennzeichnet,
daß man 40 bis 500 Gewichtsteile Verdünnungsmittel, bezogen auf 100 Gewichtsteile 2-Tert.butylphenol, und Isobuten in einem Überschuß von 1 bis 5 Mol pro Mol 2-Tert.butylphenol zusetzt.

6. Verfahren nach den Ansprüchen 1 und 5,
dadurch gekennzeichnet,
daß man 60 bis 200 Gewichtsteile Verdünnungsmittel, bezogen auf 100 Gewichtsteile 2-Tert.butylphenol, und Isobuten in einem Überschuß von 1,1 bis 2,5 Mol pro Mol Tert.butylphenol zusetzt.

7. Verfahren nach Anspruch 1 und 3,
dadurch gekennzeichnet,
daß die Reaktion in Gegenwart eines Isobutenüberschusses von 2,2 bis 5 Mol, bezogen auf 1 Mol 2-Tert.butylphenol, durchgeführt wird.

8. Verfahren nach Anspruch 1,
dadurch gekennzeichnet,
daß man die Alkylierung bei Reaktionstemperaturen von 5 °C bis 50 °C und bei Drucken von 0,2 bar bis 6 bar durchführt.

9. Verfahren nach den Ansprüchen 1 und 8,
dadurch gekennzeichnet,
daß man die Alkylierung bei Reaktionstemperaturen von 10 °C bis 20 °C durchführt.

10. Verfahren nach den Ansprüchen 1 und 8,
dadurch gekennzeichnet,
daß man die Alkylierung bei Drucken von 0,5 bis 2,5 bar durchführt.

11. Verfahren nach Anspruch 1,
dadurch gekennzeichnet,
daß man Aluminium-tris-(2-tert.butylphenolat) in Mengen von 0,05 bis 0,8 mol-%, bezogen auf eingesetztes 2-Tert.butylphenol, als Katalysator verwendet.

12. Verfahren nach den Ansprüchen 1 und 11,
dadurch gekennzeichnet,

EP 0 438 641 A2

daß man Aluminium-tris-(2-tert.butylphenolat) in Mengen von 0,1 bis 0,5 mol.-%, bezogen auf eingesetztes 2-Tert.butylphenol, als Katalysator verwendet.